# EUROPEAN PATENT APPLICATION

(11) **EP 0 958 808 A2**
(43) Date of publication of application: **24.11.1999**
(21) Application number: 98113509.8
(22) Date of filing: 20.07.1998
(51) Int. Cl.: A61K 7/15

(54) **Composition and method for inhibiting unwanted hair growth**

(30) Priority: 19.05.1998 US 81103
(71) Applicant: OTZMA CHEMISTRY LABORATORIES FOR RESEARCH AND DEVELOPMENT LTD., Tel Aviv 63291 (IL)
(72) Inventor: Bernstein, Michael, 61211 Tel Aviv (IL)
(74) Representative: VOSSIUS & PARTNER

(57) **Abstract**

A topical composition useful for inhibiting undesired hair growth, particularly following hair removal is provided. The composition comprises 1-10% (w/w) of a capsicum-derived composition of matter. The composition preferably comprises also one or more of a sage oil or aqueous sage extract, papain, anis oil and carboxylic acid. Also is provided a method for removal of undesired hair or for inhibiting undesired hair growth comprising applying the composition on the skin.

## Description

The present invention is generally in the field of cosmetics and provides a composition for inhibiting unwanted hair growth. Also provided by the invention is a cosmetic procedure for inhibiting unwanted hair growth making use of such a composition. The term "*unwanted hair growth*" means to denote growth of hair on various parts of the body, e.g. facial hair growth in women, excessive hair growth on arms, legs, chest, etc., in both men and women.

Hirsutism, or undesired excessive hair growth, is a phenomena of hormonal origin. It occurs in both women and men and may be of genetic origin or may result from hormonal, age-related changes. An example of such undesired hair growth, particularly in women, is growth of facial hair, e.g. above the upper lip, in the temples or on the chin, excessive hair growth on arms and legs, etc. Women who suffer from hirsutism have to undergo occasional cosmetic treatments to remove such undesired hair.

Removal of undesired hair growth may be carried out by the use of depilators or shaving apparatuses, by means of a hair epilating wax which is applied on the skin and when removed, plucks out hair which become attached thereto. However, depilation or hair removal in fact stimulates growth of hair and soon after the treatment hair regrows and has to be removed again.

Preparations which would inhibit regrowth of hair following depilation are thus highly desired in the cosmetic industry.

The concentration of ingredients, in the text below, is given in percent, meaning weight units of ingredients per 100 weight units of ingredients. For example, a 1% concentration means 1 gram of respective ingredient in a 100 gram total weight of a composition.

The present invention provides a topical cosmetic composition, particularly useful for inhibiting undesired hair growth, comprising, as an active ingredient 1-10% (w/w) capsicum-derived compositions of matter.

The above concentration of the capsicum-derived composition of matter is based on the dry weight of this ingredient.

Capsicum is a genus of shrubby plants of the family Solanaceae. Included in this genus are bird pepper, cayenne pepper, hot pepper, sweet pepper and others. The capsicum-derived preparation, may be ground dried capsicum fruit or seed, an aqueous or organic extract of such fruits or seeds, etc.

The term "*capsicum*" will at times be used hereinbelow to denote (in short) the capsicum-derived composition of matter.

The composition of the invention may be an aqueous composition comprising aqueous or essentially aqueous ingredients. Alternatively, the composition may also incorporate non-aqueous ingredients in which case the composition will be an oily composition or an aqueous composition comprising oily ingredients, e.g. in the form of an emulsion. The term "*aqueous composition*" will be used to denote a composition of the invention which is essentially comprised of aqueous ingredients, i.e. all or most ingredients (e.g. more than 99% or typically more than 99.5%) being water soluble. The term "*oily composition*" will be used to denote a composition comprising oily ingredients. An aqueous composition of the invention will typically be in the form of a gel. An oily composition of the invention will typically be in the form of a cream, salve or ointment, or will be a combined gel/cream composition.

In accordance with a preferred embodiment of the invention, the composition comprises also one or more of the following auxiliary active ingredients:
i. Sage oil or aqueous sage extract. Sage is a plant of the genus salvia and includes plants such as blue sage, clary sage, scralage sage. The oil or the extract are obtained from the leaves or the flowers of this plant. The sage oil contains essential oils such as that obtained from the leaves of the common sage (salvia officinalis), or an oil obtained from flowers of clary (salvia sclarea) (i.e. clary sage oil). The concentration the aqueous sage extract (based on a dry weight of this ingredient) or sage oil is typically within the range of about 0.5-25%, preferably about 3%;
ii. Papain, typically provided within an aqueous extract of non-ripe (green) papaya fruits or within an aqueous extract of papaya leaves. The concentration of this extract (based on its dry weight) is typically within the range of about 1-30%, preferably about 10%;
iii. Anis oil, typically within the range of about 0.1-10%, preferably about 0.20%;
iv. One or more carboxylic acids, which may for example be fruit acids, e.g. glycolic, citric, lactic, or ascorbic acid, typically within a concentration range of about 0.2-20%, preferably about 2.3%.

In accordance with a particular preferred embodiment of the invention, the composition comprises all of the above four auxiliary active ingredients (i)-(iv).

As will be appreciated, the composition of the invention may also comprise a variety of cosmetically acceptable carriers or diluents (e.g. ethyl alcohol), may comprise preserving agents, emulsifiers, galling agents, anti-oxidants, vitamins, minerals, astringent agents (natural and non-natural), etc.

Also provided by the invention is a cosmetic method, for removal of undesired hair, comprising removal of undesired hair off a skin portion and applying the composition of the invention onto the skin portion either prior or after the hair removal. Still further provided by the invention is a method for inhibiting growth of undesired hair, particularly, but not limited to, following removal of hair.

In a hair removal method in accordance with the invention, the composition of the invention may be applied as a prior treatment shortly before hair removal, for example, 10-30 minutes prior to the epilation; it may be applied, as an after treatment, following hair removal, for example, immediately after or at periodical intervals thereafter, e.g. once daily; or it may be applied both before or after hair removal.

Preferably, for the prior treatment in accordance with the invention use is made with the aqueous composition, and for the after treatment use is made with the oil composition of the invention.

In accordance with the preferred embodiment, the method comprises:
(a) applying a composition of the invention on a portion of the skin from which hair is to be removed, typically an aqueous, gel composition;
(b) removing the hair from said skin portion; and
(c) applying a composition of the invention, typically an oily composition but possibly also an aqueous composition of the invention or said skin portion, the composition being applied in a single application immediately after treatment or periodically, e.g. once daily.

The hair removal in the above method may be by epilation using any one of known epilation devices; it may be by shaving; it may be by the use of various hair removal preparations, e.g. wax; etc.

The application of the composition on the skin is typically in combination with heating of the skin. The heating of the skin may be either prior or during application of the composition. It was found, that such heating improves penetration to the skin presumably through pores which open up in the skin during heating. The heating may be by means of an infrared lamp, by a contact heater (a heater comprising a probe or a heating surface, which is brought into contact with the skin), by means of hot water (e.g. a shower or a bath), etc.

The invention will now be described in the following non-limiting examples.

### Example 1:

### A typical composition of the Invention and its preparation

A typical composition of the invention comprises the following ingredients:

| **No.** | **Ingredient** | **Concentration (%)** |
|---|---|---|
| 1. | Deionized water | 56.65 |
| 2. | Sage extract | 3.00 |
| 3. | Allntoin | 0.50 |
| 4. | Peg 400 - polyethylene glycol | 1.00 |
| 5. | Tween 20 | 1.50 |
| 6. | Cetyl alcohol | 2.50 |
| 7. | Lanete Sx | 2.00 |
| 8. | Isopropyl myristate | 2.00 |
| 9. | Imidazole urea (or a comparable preservative) | 0.2 |
| 10. | Bronopol | 0.10 |
| 11. | Lecitin | 2.00 |
| 12. | Kammomite extract | 3.00 |
| 13. | Capsicum extract | 10.00 |
| 14. | Carbopol 940 | 0.05 |
| 15. | Papaya extract (aqueous extract of papaya fruit or leaves) | 10.00 |
| 16. | Triethanol amine | 1.00 |
| 17. | Ascorbic acid | 0.20 |
| 18. | Lactic acid | 1.00 |
| 19. | Glicolc acid | 1.00 |
| 20. | Clove oil | 0.20 |
| 21. | Anis oil | 0.20 |
| 22. | Sage oil | 0.50 |
| 23. | Camphor oil | 0.20 |
| 24. | Lemon oil | 0.20 |
| 25. | Geranium oil | 0.30 |
| 26. | Sandalwood oil | 0.10 |
| 27. | Clary sage oil | 0.10 |
| 28. | Eucalyptus oil | 0.10 |
| 29. | Methyl paraben | 0.05 |
| 30. | Propyl paraben | 0.05 |
| 31. | Fragrance | 0.30 |
| Total | | 100 |

As will no doubt be appreciated, the above ingredients are exemplary only and some of the listed ingredients may be deleted, some may be replaced by others, and ingredients not mentioned herein may be added, e.g. a variety of ingredients known *per se* in the cosmetics industry. Furthermore, also the concentrations of the ingredients are exemplary and the concentration of each ingredient may vary.

The composition may be prepared as follows:

Ingredient number 1-8 are added together into a vessel, e.g. a stainless steel vessel, and the mixture is heated to about 85°C. The mixture is rapidly mixed and while mixing, ingredients 9-14 are added. The mixing is continued for about 30 minutes, until homogeneity.

After the carbopol gel and the remaining ingredients have become fully dissolved, triethanol amine (ingredient no. 16) is added until reaching a pH of 6.5. The mixture is then cooled to about 65°C and the papaya extract (ingredient 15) is then added. Then the fruit acids (ingredients 17-19) are added until the pH reaches about 5.5 and the temperature is cooled further to 55°C.

The essential oils (ingredients 20-28) are mixed in a separate vessel and are then added to the forming gel while continuing mixing until yielding a homogenic gel/cream; the temperature is maintained at about 55°C during this mixing stage. Then the preservatives (ingredients 29 and 30) and the fragrance (ingredient 31) are added and the composition is allowed to cool to room temperature.

A composition comprising ingredients 1-19 is essentially an aqueous composition and may be combined with the preservatives (ingredients 29 and 30) and the fragrance (ingredient 31) to yield an aqueous composition which may be used as such. Combining the remaining ingredients (20-28) yields an oily composition of the invention.

### Example 2:

### Manner of use

(a) Pretreatment: A composition of the invention is applied on the skin's surface which is to be subject to epilation for about 10-20 minutes. This composition is preferably the aqueous composition, e.g. that described in Example 1. The skin is then heated, which healing is typically accompanied by massaging of the skin so as to improve penetration of the composition to the skin. Heating may be by the use of infrared light or by contact heater, i.e. a device intended for local heating of the skin by contact therewith.
   After sufficient time to allow absorption of the active ingredients by the skin, the remainder of the oily components of the compositions (if such were included in the composition), which remain of the skin, are removed by the use of alcohol or medicinal benzene formulation, until complete cleaning of the surface.
(b) Hair removal:A hot or cold epilating wax preparation is applied and the hairs are plucked by removal of the wax, as known *per se*. Alternatively, hair removal may also be performed by an epilating or shaving device.
(c) After treatment: After this hair removal step, the oily composition, e.g. the gel/cream composition of Example 1 is applied again for about 10-20 minutes while heating and massaging similarly as in (a). The gel/cream composition is then applied once daily for 4-5 days, preferably after heating the skin, e.g. following a hot bath.

### Example 3:

### Experimental results

The following is a description of four experiments, within the framework of the present invention, carried out on women. In each experiment, the number of hairs in a specific tested area of the skin was counted both prior and after treatment by the use of a magnifying glass. This count was compared to control, i.e. hair removal without treatment. In these experiments the hair was removed from women's faces, legs and other body parts.

The summary of the experiments and the results are as follows;

### Experiment 1

A composition of the invention was applied on to treated women's leg skin prior to epilation and hair was plucked as described in Example 2.

This treatment resulted in about 1-2 months delay in hair regrowth as compared to hair removal treatment without applying composition of the invention (prior art treatment); namely, in prior art treatment, hair regrows after about 14 days, whereas in this treatment hair regrowth was observed only after about 60 days. In addition, on treated legs the regrowth hair was less dense. The hair was thinner and faker.

### Experiment 2

Composition of the invention was applied after epilation and in this case there was a delay of about 3 months in hair regrowth as compared to prior art treatment. Furthermore, in this case, the extent of hair growth (counted by the number of hairs) was only about 50-60% as compared to that observed after the prior art treatment, and the hairs which grew were relatively small and thin.

### Experiment 3

A gel composition was first applied prior to epilation and then immediately after epilation a gel/cream composition was applied. In 70% of the treated women, hair did not grow at all, and in 30% of the women there was a delay of about 2 months in hair growth as observed after prior art treatment. Furthermore, the regrown hair had a much lower density and hair was smaller and thinner.

### Experiment 4

Treatment was performed as in 3 and furthermore the women were instructed to continue with 4-5 daily applications of preparation. In 70% of the women, there was no hair growth and in the remainder 30% some hair growth was observed, at a density of 20% of that of the control prior art treatment.

## Claims

1. A topical cosmetic composition comprising 1-10% (w/w) of a capsicum-derived composition of matter.

2. A composition according to Claim 1, comprising one or more of the group consisting of:
i. sage oil or aqueous sage extract;
ii. papain;
iii. anis oil; and
iv. carboxylic acids.

3. A composition according to Claim 2, wherein the sage oil is sage essential oil.

4. A composition according to Claim 2, wherein the carboxylic acids are fruit acids.

5. A composition according to Claim 2, comprising all of the four ingredients (i)-(iv).

6. Use of a composition as defined in any one of Claims 1-5, for inhibiting undesired hair growth.

7. A cosmetic method comprising removing the hair from a portion of a skin and applying on said portion a composition according to any one of Claims 1-5, either prior or after depilation.

8. A method according to Claim 7, comprising:
(a) applying a composition comprising 1-10% (w/w) of a capsicum-derived composition of matter on a portion of the skin from which hair is to be removed;
(b) removing the hair from the skin portion; and
(c) applying said composition on said skin portion.

9. A method according to Claim 7, wherein the skin is heated prior to or during the application of said composition.

10. A method according to Claim 8, wherein the skin is heated prior to or during the application of said composition.

11. A method for inhibiting growth of undesired hair on a portion of the skin, comprising applying on said portion a composition according to any one of Claims 1 to 5.

12. A method according to Claim 11, wherein said portion is a portion from which hair has been removed.
